# EUROPEAN PATENT APPLICATION

(11) **EP 3 449 947 A1**
(43) Date of publication of application: **06.03.2019**
(21) Application number: 17204052.9
(22) Date of filing: 28.11.2017
(51) Int. Cl.: A61L 2/10, D06F 58/00, D06F 25/00

(54) **A STERILIZATION SYSTEM AND A DOOR COMPRISING THE SAME**

(30) Priority: 28.08.2017 TR 201712875
(71) Applicant: Vestel Beyaz Esya Sanayi ve Ticaret A.S., 45030 Manisa (TR)
(72) Inventor: CALISKAN, Sinan, 45030 Manisa (TR); AKTAS, Serhat, 45030 Manisa (TR); UGURLU, Murat, 45030 Manisa (TR)
(74) Representative: Cayli, Hülya

(57) **Abstract**

The sterilization system developed according to the present invention is suitable to use for sterilization of textile products by destroying the microorganisms that cannot be killed during cleaning/drying processes in a washing/drying device comprising at least one compartment which has at least one loading opening for receiving the textile product and in which washing/drying operations for a textile product is performed, and a door which has an open position for enabling access to the loading opening and a closed position for preventing access to the loading opening and which comprises, on the side facing the compartment when the door is in the closed position, a main body (K1) having a region (K2) with luminous transmittance at the ultraviolet wavelength, wherein the sterilization system comprises at least one body (1), which comprises at least a first slot (1a) having at least one opening and which is adapted to be positioned such that the opening faces the compartment from said region (K2) on the main body (K1); at least one ultraviolet light source (2), which emits light at the ultraviolet wavelength that allows destroying microorganisms, and which is positioned inside the first slot (1a) such that it ensures that UVC light is transmitted onto the textile products in the compartment from the opening; and at least one insulating element (4), which provides insulation between the main body (K1) and the body (1), and which is positioned around the opening.

## Description

### Field of Invention

The present invention relates to sterilization systems used in washing devices and to doors having such systems.

### Background of the Invention

For the cleaning/drying of a textile product, washing/drying devices comprising a compartment, in which cleaning/drying operation is performed, are used. Such washing/drying devices comprise a door for controlling the access to the compartment, and they may be a top-loading or front-loading devices based on the position of the door. According to the cleaning processes included in the washing/drying device, the textile products are cleaned with various cleaning materials. However, these cleaning processes may not be sufficient for sterilization of the products, and thus different sterilization applications are required for especially cleaning baby products or the products belonging to users with allergic conditions. Within this context, in prior art, the systems that emit light at the ultraviolet wavelength can be used, whereby it can be ensured that the microorganisms which may be present on the products after the cleaning/drying process are destroyed.

An example of the systems emitting light at the ultraviolet wavelength is disclosed in the patent document no. EP2113605B1. In the document, there is disclosed a household appliance for cleaning a textile product, wherein the household appliance comprises a washing compartment and an ultraviolet light source that emits light at the ultraviolet wavelength into the washing compartment. With the light emitted by the light source, the photocatalytic chemical reactions are activated so as to remove the stains on the textile product. However, in order to be able to use the application disclosed in said document, the textile products must have photocatalytic properties. Therefore, the system disclosed in said document cannot be used for all the textile products, and thus it cannot completely eliminate the problem concerning sterilization.

### Brief Description of the Invention

The sterilization system developed according to the present invention is suitable to use for sterilization of textile products by destroying the microorganisms that cannot be killed during cleaning/drying processes in a washing/drying device comprising at least one compartment which has at least one loading opening for receiving the textile product and in which washing/drying operations for a textile product is performed, and at least one door which has an open position for enabling access to the loading opening and a closed position for preventing access to the loading opening and which comprises, on the side facing the compartment when the door is in the closed position, at least one main body having at least one region with luminous transmittance at the ultraviolet wavelength, wherein the sterilization system comprises at least one body, which comprises at least a first slot having at least one opening and which is adapted to be positioned such that the opening faces the compartment from said region on the main body; at least one ultraviolet light source, which emits light at the ultraviolet wavelength that allows destroying microorganisms, and which is positioned inside the first slot such that it ensures that UVC light is transmitted onto the textile products in the compartment from the opening; and at least one insulating element, which provides insulation between the main body and the body, and which is positioned around the opening.

The door developed according to the present invention is also suitable to use for controlling the access to the loading opening in a washing/drying device comprising at least one compartment which has at least one loading opening for receiving the textile product and in which washing/drying operations for a textile product is performed, wherein the door has an open position for enabling access to the loading opening and a closed position for preventing access to the loading opening and comprises at least one main body; at least one region with luminous transmittance at the ultraviolet wavelength, which is provided on the side of the main body that faces the compartment when the door is in the closed position; and at least one said sterilization system which is positioned on the main body such that the light at the UVC wavelength emitted by the ultraviolet light source comprised by said system passes through said region so as to reach the laundries inside the compartment of the washing/drying device.

By virtue of the sterilization system of the present invention and the door having this system, it is ensured that the microorganisms, which may be present on the laundries after the cleaning/drying process thereof, are effectively destroyed, in particular in front-loading washing/drying devices, and thus sterilization conditions are able to be met especially for the baby products and/or the products belonging to users with allergic conditions.

### Object of the Invention

An object of the present invention is to provide a sterilization system which is suitable to use especially in front-loading washing/drying devices, and a door having said system.

Another object of the present invention is to provide a sterilization system in which sterilization process is performed by emitting light at the ultraviolet wavelength, and a door having said system.

Yet another object of the present invention is to provide a sterilization system that is able to be used in sterilization of all the textile products, and a door having said system.

Still another object of the invention is to provide a sterilization system allowing for performing the sterilization process effectively, and a door having said system.

### Description of the Drawings

Exemplary embodiments of the sterilization system developed according to the present invention and also of the door having said system are illustrated in the attached drawings, wherein:
Figure 1 is an exploded view of the sterilization system developed according to the present invention.
Figure 2 is an exploded view of the use of the sterilization system developed according to the present invention in a door;
Figure 3 is an exploded view of the sterilization system and the door according to the present invention.

All the parts illustrated in the drawings are individually assigned a reference numeral and the corresponding terms of these numbers are listed as follows:

| | |
|---|---|
| Main body | (K1) |
| Region | (K2) |
| Second slot | (K3) |
| Third slot | (K4) |
| Main cover | (K5) |
| Body | (1) |
| First slot | (1a) |
| Hole | (1b) |
| Ultraviolet light source | (2) |
| Cover | (3) |
| Insulating element | (4) |
| Casing | (5) |
| Indicator | (6) |
| Protective cover | (7) |

### Description of the Invention

In the washing/drying devices used for cleaning/drying a textile product, there is provided a compartment in which washing/drying operations for a textile product is performed, and a loading opening for having access to said compartment. The access to the loading opening is provided by a door comprised by the washing/drying device. The door is in a closed position during cleaning/drying process, and when said process is over, the door is opened so as to provide access to the cleaned/dried textile products. The textile products may not be sufficiently sterilized even after the washing/drying operations performed in washing/drying devices, which may sometimes result in unfavourable situations for those who use these textile products. For example, if said textile product belongs to a user with allergic conditions, then it may cause allergic reactions in the user. Therefore, with the present invention, there is provided a sterilization system, which is suitable to use in washing/drying devices and ensures sterilization of textile products by destroying the microorganisms that cannot be killed during cleaning/drying processes, and a door having said system.

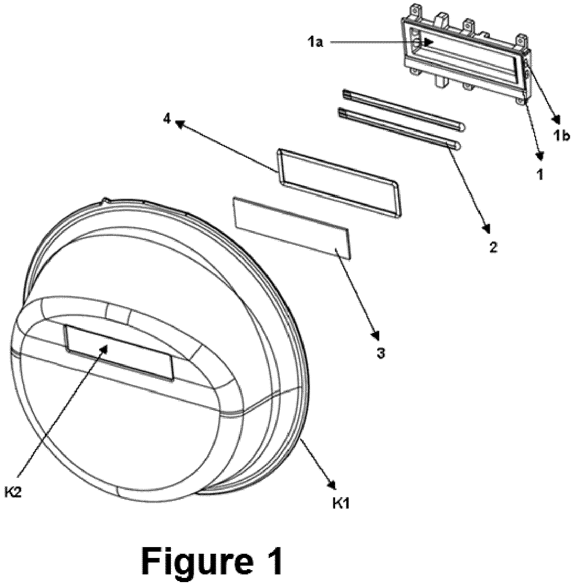

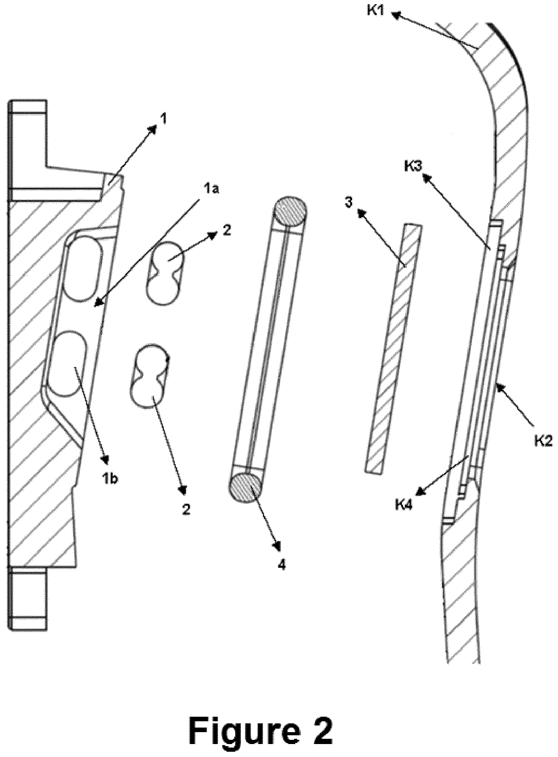

The sterilization system of the present invention, the exemplary views of which are illustrated in Figures 1-3, is suitable to use for sterilization of textile products by destroying the microorganisms that cannot be killed during cleaning/drying processes preferably in a front-loading washing/drying device comprising at least one compartment, which has at least one loading opening for receiving the textile product and in which washing/drying operations for a textile product is performed, and at least one door which has an open position for enabling access to the loading opening and a closed position for preventing access to the loading opening and which comprises, on the side facing the compartment when the door is in the closed position, at least one main body (K1), preferably at least some part of which extends into the compartment and has at least one region (K2) with luminous transmittance at the ultraviolet wavelength, wherein the sterilization system comprises at least one body (1), which comprises at least a first slot (1 a) having at least one opening and which is adapted to be positioned such that the opening faces the compartment from said region (K2) on the main body (K1); at least one ultraviolet light source (2), which emits light at the ultraviolet wavelength (UVC) that allows destroying microorganisms, and which is positioned inside the first slot (1a) such that it ensures that UVC light is transmitted onto the textile products in the compartment from the opening; and at least one insulating element (4), preferably in the form of a frame, which provides insulation between the main body (K1) and the body (1), and which is positioned around the opening.

In an exemplary embodiment of the invention, the ultraviolet light source (2) is positioned in the first slot (1 a), and the body (1) is positioned on the main body (K1) such that UVC light is ensured to reach into the compartment from the region (K2) on the side of door that faces the compartment when the door is in its closed position. When the sterilization operation is initiated in the washing/drying device, the ultraviolet light source is energized via a control unit to emit UVC light. The UVC light reaches from the opening to said region (K2), therefrom passes into the compartment so as to reach the textile products in the compartment. Thus, it is ensured that the microorganisms that are present on the textile products are destroyed, and an effective and safe sterilization system is achieved.

In a preferred embodiment of the present invention, the sterilization system comprises at least one cover (3), which is made of a UVC light transmitting material (e.g. quartz glass) and which covers said opening. Said cover (3) is preferably positioned on the opening such that the cover will be surrounded by the insulating element (4), thereby an effective and safe sterilization system is achieved.

In another alternative embodiment of the present invention, the sterilization system preferably comprises at least two holes (1 b) which are provided on the opposite side walls of the body (1), and which are positioned in opposition to each other such that at least one hole is provided on each wall. In this embodiment, the ultraviolet light source (2) is preferably in the form of a bar, and is positioned inside the first slot (1 a) by passing, preferably sliding, through one of the holes (1b) towards the other. Thus, the ultraviolet light source (2) is able to be fixed on the body (1) in a practical and reliable manner.

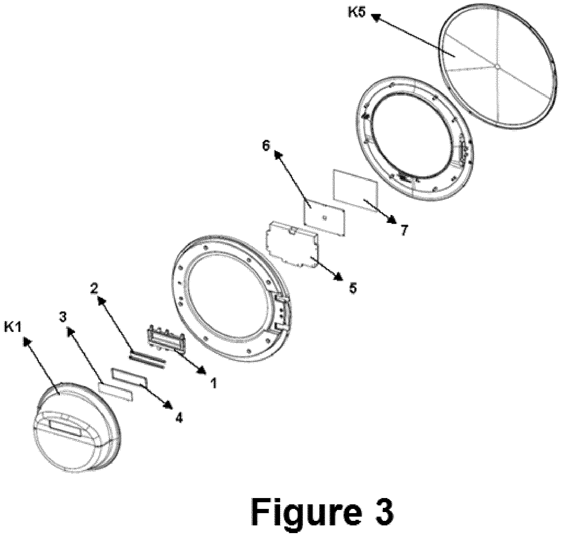

In another preferred embodiment of the present invention, the sterilization system comprises at least one warning assembly which provides the user with information about sterilization process in terms of user safety. As shown in Figure 3, said warning assembly preferably comprises at least one casing (5) provided on the side of said body (1) that does not have opening; at least one indicator (6) which is positioned within said casing (5) and on which there is provided a plurality of light sources (e.g. LED) for transmitting the information message to the user; and at least one control assembly which is in connection with the control unit of the washing/drying device, and which receives data relating to the sterilization process (e.g. sterilization is active or sterilization is over, etc.) from the control unit and communicates it to the user via the indicator (6). With this embodiment, the user is informed about the sterilization process, and a reliable sterilization system is achieved by preventing any possible harm to the user. In this embodiment, the sterilization system of the present invention further comprises at least one protective cover (7) which is provided such that the indicator (6) is positioned between the protective cover and the casing (5). Thus, by virtue of protecting the indicator (6) against external factors, a long-lasting sterilization system is achieved.

Still in another preferred embodiment of the invention, said ultraviolet light source (2) is positioned so as to make preferably an angle of between 0 to 15 degrees with respect to the normal of the ground on which the washing/drying device is located. In this manner, it is ensured that UVC light reaches all the surfaces of the laundries in the compartment, whereby an effective sterilization system is achieved.

The door developed according to the present invention is also suitable to use for controlling the access to the loading opening in a washing/drying device comprising at least one compartment which has at least one loading opening for receiving the textile product and in which washing/drying operations for a textile product is performed, wherein the door has an open position for enabling access to the loading opening and a closed position for preventing access to the loading opening and preferably comprises at least one main body (K1), preferably at least some part of which extends into the compartment. The door according to the present invention also comprises at least one region (K2) with luminous transmittance at the ultraviolet wavelength, which is provided on the side of the main body (K1) that faces the compartment when the door is in the closed position; and at least one said sterilization system which is positioned on the main body (K1) (preferably on the side of the main body (K1) not facing the compartment) such that the light at the UVC wavelength emitted by the ultraviolet light source (2) comprised by said system passes through said region (K2) so as to reach the laundries inside the compartment of the washing/drying device.

In a preferred embodiment of the invention, the door comprises at least a second slot (K3) which is positioned on the main body (K1) so as to be on the side of the region (K2) not facing the compartment when the door is in its closed position, and which is suitable for receiving the insulating element (4) of the sterilization system. Thus, by virtue of fixing the sterilization system to the door properly, a reliable door is achieved. The door developed according to this embodiment further comprises at least a third slot (K4), preferably with a smaller size than the second slot (K3), and positioned between the second slot (K3) and said region (K2). The third slot (K4) receives the cover (3) comprised by the sterilization system.

In a further alternative embodiment, the main body (K1) and the body (1) are made of plastic and are fastened to each other by ultrasonic welding, vibration welding and / or hot plate welding.

In a further exemplary embodiment, the door preferably comprises at least one main cover (K5) which is positioned on the side of the main body (K1) not facing the compartment when the door is in the closed position such that the sterilization system will be between the main body (K1) and the main cover. Said main cover (K5) is preferably made of a material that enables the user to see the communications provided to the user via the indicator comprised in the warning assembly of the sterilization system.

By virtue of the sterilization system of the present invention and the door having this system, it is ensured that the microorganisms, which may be present on the laundries after the cleaning/drying process thereof, are effectively destroyed, in particular in front-loading washing/drying devices, and thus the required sterilization conditions are able to be met especially for the baby products and/or the products belonging to users with allergic conditions.

## Claims

1. A sterilization system, suitable to use for sterilization of textile products by destroying the microorganisms that cannot be killed during cleaning/drying processes in a washing/drying device comprising at least one compartment which has at least one loading opening for receiving the textile product and in which washing/drying operations for a textile product is performed, and at least one door which has an open position for enabling access to the loading opening and a closed position for preventing access to the loading opening and which comprises, on the side facing the compartment when the door is in the closed position, at least one main body (K1) having at least one region (K2) with luminous transmittance at the ultraviolet wavelength, the sterilization system being **characterized by** comprising:
- at least one body (1), which comprises at least a first slot (1 a) having at least one opening and which is adapted to be positioned such that the opening faces the compartment from said region (K2) on the main body (K1);
- at least one ultraviolet light source (2), which emits light at the ultraviolet wavelength (UVC) that allows destroying microorganisms, and which is positioned inside the first slot (1a) such that it ensures that UVC light is transmitted onto the textile products in the compartment from the opening; and
- at least one insulating element (4) which provides insulation between the main body (K1) and the body (1), and which is positioned around the opening.

2. A sterilization system according to Claim 1, **characterized in that** said insulating element (4) is in the form of a frame.

3. A sterilization system according to Claim 1, **characterized by** comprising at least one cover (3) which is made of a UVC light transmitting material and covers said opening.

4. A sterilization system according to Claim 3, **characterized in that** the cover (3) is made of quartz glass.

5. A sterilization system according to Claim 3, **characterized in that** said cover (3) is positioned on the opening such that the cover will be surrounded by the insulating element (4).

6. A sterilization system according to Claim 1, **characterized by** comprising at least two holes (1 b) which are provided on the opposite side walls of the body (1), and which are positioned in opposition to each other such that at least one hole is provided on each wall.

7. A sterilization system according to Claim 1, **characterized by** comprising at least one warning assembly for informing the user about the sterilization process.

8. A sterilization system according to Claim 7, **characterized in that** the warning assembly comprises at least one casing (5) provided on the side of said body (1) that does not have opening; at least one indicator (6) which is positioned within said casing (5) and on which there is provided a plurality of light sources for transmitting the information message to the user; and at least one control assembly which is in connection with the control unit of the washing/drying device, and which receives data relating to the sterilization process from the control unit and communicates it to the user via the indicator (6).

9. A sterilization system according to Claim 8, **characterized by** comprising at least one protective cover (7) which is provided such that the indicator (6) is positioned between the protective cover and the casing (5).

10. A sterilization system according to any one of the preceding claims, **characterized in that** ultraviolet light source (2) is positioned in the first slot (1a) so as to make preferably an angle of between 0 to 15 degrees with respect to the normal of the ground on which the washing/drying device is located.

11. A door, suitable to use for controlling the access to the loading opening in a washing/drying device comprising at least one compartment which has at least one loading opening for receiving the textile product and in which washing/drying operations for a textile product is performed, wherein the door has an open position for enabling access to the loading opening and a closed position for preventing access to the loading opening and comprises at least one main body (K1), the door being **characterized by** comprising:
- at least one region (K2) with luminous transmittance at the ultraviolet wavelength, which is provided on the side of the main body (K1) that faces the compartment when the door is in the closed position; and
- at least one sterilization system according to any of the preceding claims, which is positioned on the main body (K1) such that the light at the UVC wavelength emitted by the ultraviolet light source (2) comprised by said system passes through said region (K2) so as to reach the laundries inside the compartment of the washing/drying device.

12. A door according to Claim 11, **characterized in that** the structure of the main body (K1) is such that at least some part of which extends into the compartment.

13. A door according to Claim 11, **characterized by** comprising at least a second slot (K3) which is positioned on the main body (K1) so as to be on the side of the region (K2) not facing the compartment when the door is in its closed position, and which is suitable for receiving the insulating element (4) of the sterilization system.

14. A door according to Claim 11, **characterized by** comprising at least one main cover (K5) which is positioned on the side of the main body (K1) not facing the compartment when the door is in the closed position such that the sterilization system will be between the main body (K1) and the main cover.

15. A door according to Claim 14, **characterized in that** the main cover (K5) is made of a material that enables the user to see the communications provided to the user via the indicator comprised in the warning assembly of the sterilization system.
